# EUROPEAN PATENT APPLICATION

(11) **EP 4 509 666 A1**
(43) Date of publication of application: **19.02.2025**
(21) Application number: 24190944.9
(22) Date of filing: 25.07.2024
(51) Int. Cl.: E03D 13/00

(54) **SYSTEMS FOR HUMAN WASTE COLLECTION AND CONTAINMENT**

(30) Priority: 02.08.2023 US 202318364084
(71) Applicant: Hamilton Sundstrand Space Systems International, Inc., Windsor Locks, CT 06096 (US)
(72) Inventor: RADAWIEC, Rochelle, Vernon, CT 06066 (US); LUKER, Kelly, Glastonbury, CT 06033 (US)
(74) Representative: Dehns

(57) **Abstract**

Waste management systems and capture funnels for use therewith are described. The waste management systems include a waste container (104), a reclamation system (118), a capture funnel (102), and a hose (106) fluidly connecting the capture funnel to each of the waste container and the reclamation system. The capture funnels include a funnel having an inlet end (212) and an outlet end (308), a water-function trigger (214) actuatable to dispense water into the funnel, and an air-function trigger (216) actuatable to dispense air into the funnel.

## Description

### BACKGROUND

The subject matter disclosed herein generally relates to systems and mechanisms for collection and containment of human waste and, more particularly, to improved funnel and collection devices related thereto.

When humans reside in relatively closed spaces or environments (e.g., on ships, aircraft, spacecraft, or the like), or in remote locations or the like, waste management (e.g., of human waste including excrement) must be managed to avoid contamination, disease, sanitation, and the like. If conventional plumbing is not required or there environmental impacts to plumbing functionality, specific or uniquely designed systems may be required to manage human waste. For example, when in low, micro, or no gravity (e.g., in space), a lack of gravity prevents conventional plumbing to be employed. Further, due to the low or no gravity, containment of liquids and solids is an important consideration.

In current systems, a hose with a funnel is connected to a waste collection system and a fan or the like is used to generate a suction force. A user (e.g., human user) will apply the funnel to the body and excrete waste (urine/menses) into the funnel, which captures the waste. The connected hose will provide a path through which the waste is pulled (or pushed) and the liquid and gaseous portions may be separated. Solid waste (and/or liquids) may be directed into a holding container or the like. In current systems, the funnels that may be used with waste management systems are expensive and may require replacement at 60-day intervals. Further, the process of urinating is messy and can generate excess or extra waste from dry and wet wipes that users employ when using the current systems. For example, the conventional funnels required being cleaned (e.g., wiped with sanitizing wipes) after each use. These wipes (dry and/or wet) will be disposed of within the same waste management system and as a result will occupy, take up, or otherwise fill volume/space in a container or containment device (e.g., toilet or receptacle). With more remote space travel, emphasis on minimizing consumable resources will become more important. Furthermore, using current systems, individuals use their own funnel, requiring additional hardware and dedicated funnels and associated components for each occupant and/or user. Accordingly, improved waste management systems are desirable to improve sanitation and living conditions.

### SUMMARY

According to one aspect, waste management systems are provided. The waste management systems include a waste container, a reclamation system, a capture funnel, and a hose fluidly connecting the capture funnel to each of the waste container and the reclamation system. The capture funnel includes a funnel having an inlet end and an outlet end, a water-function trigger actuatable to dispense water into the funnel, and an air-function trigger actuatable to dispense air into the funnel.

Embodiments of the waste management systems may include that the funnel comprises at least one airflow aperture arranged to permit gases to pass therethrough and prevent liquid from passing through the at least one airflow aperture.

Embodiments of the waste management systems may include that the funnel of the capture funnel comprises a funnel element and a stem, wherein the funnel element is configured to selectively attach and detach from the stem.

Embodiments of the waste management systems may include that the funnel element is a first funnel element and the capture funnel comprises a second funnel element, wherein the second funnel element is different in size from the first funnel element, and wherein each of the first funnel element and the second funnel element are configured to selective attach and detach from the stem.

Embodiments of the waste management systems may include a clean water source fluidly connected to the funnel by a water line, wherein actuation of the water-function trigger causes clean water from the clean water source to be dispensed into the funnel.

Embodiments of the waste management systems may include a water pump arranged along the water line and is selectively operable based on actuation of the water-function trigger.

Embodiments of the waste management systems may include a valve arranged between an end of the water line and the funnel, wherein the valve is configured to prevent backflow of liquid into the water line from the funnel.

Embodiments of the waste management systems may include an air source fluidly connected to the funnel by an air line, wherein actuation of the air-function trigger causes air from the air source to be dispensed into the funnel.

Embodiments of the waste management systems may include an air treatment module arranged along the air line and configured to treat the air prior to being dispensed into the funnel.

Embodiments of the waste management systems may include that the air treatment module comprises at least one of a heater and a dehumidifier.

Embodiments of the waste management systems may include a valve arranged between an end of the air line and the funnel, wherein the valve is configured to prevent backflow of liquid into the air line from the funnel.

Embodiments of the waste management systems may include a fluid separation junction arranged along the hose and configured to separate liquid and gas fluids passing through the hose.

Embodiments of the waste management systems may include that the waste container comprises a removable container insert.

Embodiments of the waste management systems may include that the waste management system is configured for use in a low gravity environment.

According to another aspect, capture funnels for use with waste management systems are provided. The capture funnels include a funnel having an inlet end and an outlet end, a water-function trigger actuatable to dispense water into the funnel, and an air-function trigger actuatable to dispense air into the funnel.

Embodiments of the capture funnels may include that the funnel comprises a funnel element and a stem, wherein the funnel element is configured to selectively attach and detach from the stem.

Embodiments of the capture funnels may include that the funnel element is a first funnel element and the capture funnel comprises a second funnel element, wherein the second funnel element is different in size from the first funnel element, and wherein each of the first funnel element and the second funnel element are configured to selective attach and detach from the stem.

Embodiments of the capture funnels may include a cover configured to selectively and sealing engage with the inlet end of the funnel.

Embodiments of the capture funnels may include that the cover comprises a sanitizing module configured to sanitize an interior of the funnel when the cover is engaged with the inlet end of the funnel.

Embodiments of the capture funnels may include a hose adapter at the outlet end of the funnel, wherein the hose adapter is configured to sealing connect to a hose of a waste management system.

The foregoing features and elements may be combined in various combinations without exclusivity, unless expressly indicated otherwise. These features and elements as well as the operation thereof will become more apparent in light of the following description and the accompanying drawings. It should be understood, however, that the following description and drawings are intended to be illustrative and explanatory in nature and non-limiting and the scope of the invention is defined by the claims.

### BRIEF DESCRIPTION OF THE DRAWINGS

The subject matter is particularly pointed out and distinctly claimed at the conclusion of the specification. The foregoing and other features, and advantages of the present disclosure are apparent from the following detailed description taken in conjunction with the accompanying drawings in which:
FIG. 1 is a schematic illustration of a waste management system that may incorporate embodiments of the present disclosure;
FIG. 2 is a schematic illustration of a waste management system in accordance with an embodiment of the present disclosure;
FIG. 3A is a schematic illustration of a capture funnel during use by a user;
FIG. 3B is a schematic illustration of the capture funnel of FIG. 3A during a water cleaning use of the capture funnel;
FIG. 3C is a schematic illustration of the capture funnel of FIG. 3A during an air drying use of the capture funnel;
FIG. 4A is a schematic illustration of a capture funnel in accordance with an embodiment of the present disclosure;
FIG. 4B is a schematic illustration of the capture funnel of FIG. 4B in a closed state for performing a cleaning operation of the capture funnel; and
FIG. 5 is a schematic illustration of a capture funnel in accordance with an embodiment of the present disclosure.

### DETAILED DESCRIPTION

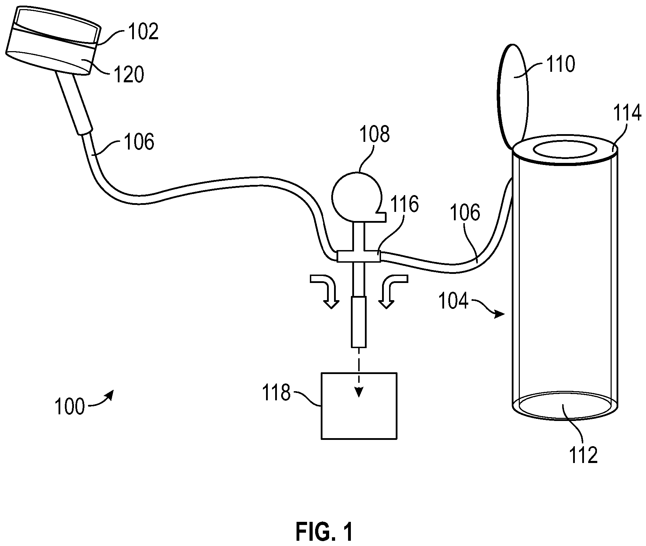

Referring to FIG. 1, a schematic diagram of a waste management system 100 in accordance with an embodiment of the present disclosure is shown. The waste management system 100 may be used onboard a ship, craft, or in a remote location. The specific described embodiments and examples of this disclosure are directed to use onboard a spacecraft or the like that is subject to low gravity, microgravity, or no gravity, when in space. However, it will be appreciated that the waste management system 100 and variations thereon, and as described herein, may be employed in other environments and/or situations, and the present description is not intended to be limited to space-based (low gravity) applications. As used herein, the term "low gravity" will be used to refer to low gravity, microgravity, and no gravity situations collectively, for ease of use and description.

The waste management system 100 includes a capture funnel 102 that is fluidly coupled to a waste container 104 by a hose 106 (e.g., hose, tube, conduit, etc.). The capture funnel 102 is configured and arranged to be applied by a user directly on the skin or with an intermediate layer (e.g., cloth layer or the like). In use, the capture funnel 102 will direct human waste (e.g., urine and/or menses) toward and into the hose 106. The hose 106 may be configured with a fluid driver 108 (e.g., pump, fan, or the like) that creates air suction and aids in directing the liquid waste into and through the hose 106. The liquid (and any solid) waste may be directed along the hose 106 to the waste container 104 and/or a reclamation system 118. For example, in some configurations, by operation of the fluid driver 108, liquid waste may be pulled from the waste container 104 and directed into the reclamation system 118.

In accordance with some embodiments, the waste container 104 may be a selectively resealable container that includes a sealable lid 110 and a removable container insert 112. The waste container 104 may be configured to receive both liquid and solid human waste. In some configurations, as noted above, the fluid driver 108 may be used to extract liquid waste from the waste container 104 and direct it to the reclamation system 118. By removal of liquid from the waste container 104, solid waste may remain for disposal using the removable container insert 112. The removable container insert 112 may be secured in the waste container 104 by the sealable lid 110 engaging with a rim 114 of the waste container 104. When the removable container insert 112 is indicated as full, the removable container insert 112 may be removed and disposed of or emptied, and a new or clean (emptied) removable container insert 112 may be installed back into the waste container 104. It will be appreciated that other configurations are possible without departing from the scope of the present disclosure. For example, in some configurations, the removable container insert 112 may not be present, and the waste container 104 may be configured to be emptied directly and sanitized, for example.

As shown, a fluid separation junction 116 may be provided along the hose 106 between the capture funnel 102 and the waste container 104. The fluid separation junction 116 may be configured to separate liquid and gaseous portions of a fluid passing through the hose 106. For example, in some configurations, a source of clean air (e.g., cabin air) may be directed into and through the capture funnel 102 and/or the hose 106 to aid in driving the fluid away from a user and toward the reclamation system 118. As shown, downstream from the fluid separation junction 116 and separate from the waste container 104 is the reclamation system 118. The reclamation system 118 may include a holding tank or storage tank and various other components or systems for treating or reclaiming both gas and liquid from the waste management system 100 (e.g., directly from a user or from the waste container 104). For example, the reclamation system 118 may be configured to receive, separate, and treat liquid waste and gas (e.g., air) from the waste management system 100. In other non-limiting configurations, air (and/or other gases) is reclaimed and may also be used in a semi-closed system for use with the waste management system 100 by a subsequent user. It will be appreciated that such air may be used for other purposes, such as for cooling, treating, thermal exchange, or the like, in one or more other systems onboard the same craft as the waste management system 100.

The capture funnel 102 may be configured for use by a human user for capture and disposal of human waste (e.g., urine). In this illustrative configuration, the capture funnel 102 may include a capture insert 120 that can prevent splashing of liquid directed into the capture funnel 102. The capture insert 120 may be a reusable or disposable insert that can absorb or direct fluids from the capture funnel 102 to the hose 106. In accordance with some embodiments of the present disclosure, the capture funnels described herein can include a funnel (e.g., funnel 102) with a wicking insert (e.g., capture insert 120) designed to more adequately direct urine into a urine hose (e.g., hose 106) of a waste management system (e.g., such as waste management system 100 of FIG. 1). In some configurations, the capture insert 120 may be positioned inside the capture funnel 102 for urine droplets to attach to in a low gravity environment. The capture insert 120 may be formed of a wicking material or the like (e.g., hydrophobic, hydrophilic, or a combination thereof depending on the specific application). In accordance with some embodiments, the material of the capture funnel 102 and/or the capture insert 120 may be anti-microbial and selected to withstand ultraviolet-light (UV) or other sanitation mechanisms (e.g., selected for use with specific sanitizing chemicals or the like). In accordance with a non-limiting example, the capture funnel 102 may be selectively removable from the hose 106. In such a configuration, each user of the waste management system 100 may provide their own capture funnel 102 that is selectively attachable to the hose 106. In some embodiments, the capture funnel 102 may include a bidet feature for cleaning of a user and/or self-cleaning of the capture funnel 102.

For example, in accordance with some embodiments of the present disclosure, a handheld funnel bidet is provided for use with waste management systems. In accordance with some embodiments, a flexible funnel is provided that fits to a user's body and doubles as the vessel for urination while providing for bidet functionality. In accordance with a non-limiting example, the capture funnel may have a shape similar to a hollow showerhead. The capture funnel may be attached to a hose of a waste management system (e.g., toilet). The capture funnel may include one or more actuators or triggers (e.g., buttons, switches, toggles, etc.) to cause operation of one or more features. For example, the capture funnel may include a water-based operation (e.g., cleaning and/or rinse operation) and an air-based operation (e.g., drying and/or removal of moisture). The different operations may be configured to be used both when applied to a human body (e.g., for urination and cleaning) and when not applied to a human body (e.g., for cleaning of the capture funnel).

In a non-limiting example of use of a capture funnel of the present disclosure, a user affixes the capture funnel to their body, urinates, and then actuates the water-function trigger (e.g., press a water-button, toggle a switch, or the like) to initiate a rinsing function. In accordance with some embodiments, the rinsing function causes clean water to be directed toward the user and thus provide a cleaning or rinsing operation of the user after urination. In accordance with some embodiments, the rinsing function may be configured to continue as long as the water-function trigger is pressed. In some embodiments, the water-function trigger can be electronically started once a liquid flow stream is no longer detected within the capture funnel. In other embodiments, the duration of the rinsing function may be based on a timer, amount of water dispensed, or based on some other criteria. The user may then actuate an air-function trigger (e.g., button, switch, toggle, etc.) to initiate a drying function. Similar to the rinsing function, the drying function may continue as long as the air-function trigger is actuated or may be operated for a preset period of time or other preset condition/duration. After performing the drying function, the user may remove or detach the capture funnel from the body.

In accordance with some embodiments, the capture funnel with handheld bidet functionality may be configured to have the cleaning feature (e.g., water and air functionality) extend the life of the capture funnel, a urine hose, and/or other components of a waste management system. For example, in some embodiments, a funnel cap may be provided that can be latched into place about an opening of the capture funnel. With the funnel cap in place, a user can initiate a cleaning sequence of the capture funnel and hose with a water stream that is then drained to a reclamation system or other holding or storage tank. The air-function trigger may also be operated to perform a drying function for the capture funnel, the hose, and other fluidly connected components (e.g., fan, pump, junctions, etc.). In some embodiments, the cleaning sequence can be started by software commands once the funnel cap is attached to the capture funnel. That is, a switch or detector/sensor may be provided on the capture funnel to register when the funnel cap is attached in the closed position and thus trigger the cleaning sequence.

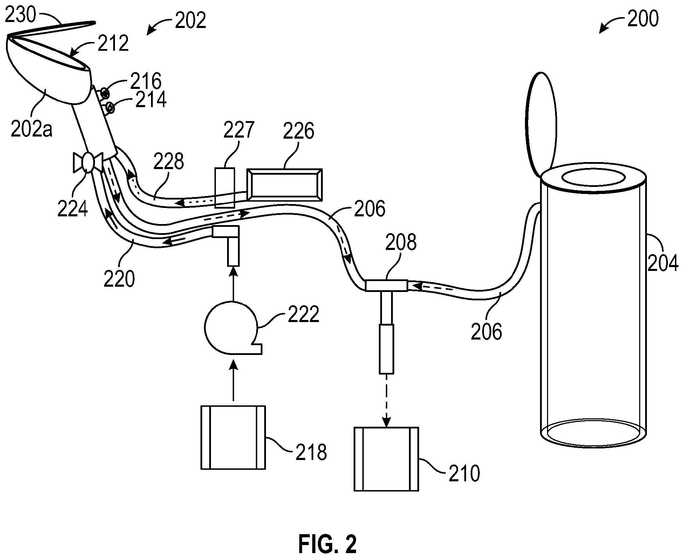

Referring now to FIG. 2, a schematic illustration of a waste management system 200 in accordance with an embodiment of the present disclosure. The waste management system 200 may be similar to the system illustrated in FIG. 1. For example, and as shown, waste management system 200 includes a capture funnel 202 that is fluidly coupled to a waste container 204 by a hose 206 (e.g., hose, tube, conduit, etc.). The capture funnel 202 is configured and arranged to be applied by a user directly on the skin or with an intermediate layer (e.g., cloth layer or the like). In use, the capture funnel 202 will direct excrement (e.g., urine) toward and into the hose 206. The capture funnel 202 includes a funnel 202a which is applied to the user for user of the system 200. The hose 206 may be configured with an optional fluid driver, such as a pump, fan, or the like, that aids in directing the liquid waste into and through the hose 206 (e.g., similar to fluid driver 108 of FIG. 1). The liquid (and any solid) waste may be directed along the hose 206 to the waste container 204 and/or a reclamation system 210, which may be arranged similar to that described above.

As shown, a fluid separation junction 208 may be provided along the hose 206 between the funnel 202a and the waste container 204. The fluid separation junction 208 may be configured to separate liquid and gaseous portions of a fluid passing through the hose 206. For example, in some configurations, a source of clean air (e.g., cabin air) may be directed into and through the funnel 202a and/or the hose 206 to aid in driving the fluid away from a user and toward the waste container 204 and/or the reclamation system 210. As illustrated, at a location downstream from the fluid separation junction 208 and separate from the waste container 204 is the reclamation system 210. The reclamation system 210 may be configured to receive, separate, and/or treat liquids and gases (e.g., urine and air) from the waste management system 200.

The capture funnel 202 is a handheld device that is used by applying an inlet end 212 (e.g., open end) of the funnel 202a to the user's body. During use, the user may urinate into the funnel 202a, and the liquid will be pulled into and through the hose 206 to the reclamation system 210. The capture funnel 202, of this embodiment, includes a bidet or cleaning feature and functionality. As shown, the capture funnel 202 includes a water-function trigger 214 and an air-function trigger 216 thereon. In accordance with some embodiments and configurations, the water-function trigger 214 and the air-function trigger 216 may be arranged on a forward or front side of the capture funnel 202 to provide for easy access and operation during use of the capture funnel 202.

The water-function trigger 214 is configured to cause dispensing of clean water from a water source 218 to be supplied through a clean water line 220 into the interior of the funnel 202a. The water-function trigger 214 may be a button, toggle, switch, knob, or the like, that may be manually operated or actuated to cause operation of a water function. In some configurations, and as shown, a water pump 222 is arranged along the clean water line 220 to dispense water into the funnel 202a. As shown, an optional valve 224 may be arranged to prevent backflow of liquid from the funnel 202a into the clean water line 220. As noted above, the dispensing of the water during a rinsing operation may be based on continuous operation of the water-function trigger 214 or based on a set duration or period after actuating the water-function trigger 214. In some configurations, the water-function trigger 214 may be operably connected to the water pump 222 (e.g., mechanically or electrically) to cause pulling water from the water source 218 and through the clean water line 220. The water may thus be used for rinsing of the body that is exposed within the funnel 202a.

After performing a rinsing operation using the water-function trigger 214, the user may performing a drying operation using the air-function trigger 216. The air-function trigger 216 may be configured to cause supplying air from an air source 226 through an air line 228. The air source 226 may be a cabin of a craft on which the waste management system 200 is employed or may be sourced from some other location (e.g., ambient location, external to the craft (if air is available), a dedicated air source container, etc.). The air source 226 may include a motive driver (e.g., such as an air pump or fan) similar to the water pump 222, although such air pump is not shown for simplicity of illustration. During the drying operation, air is directed from the air source 226, through the air line 228, and into the funnel 202a. The air may be treated using an air treatment module 227. The air treatment module 227 may be configured to treat air from the air source 226 prior to being dispensed into the funnel 202a. For example, the air treatment module may be configured to heat and/or dry the air using components or mechanisms, such as heating coils, heat exchangers, dehumidifiers, liquid-air separators, and the like, prior to dispensing into the funnel 202a. In some such embodiments, the air treatment functionality and/or components (e.g., air treatment module 227) may be integrated into the air source 226 or may be arranged along the air line 228. Similar to the clean water line 220, the air line 228 may include a valve or the like to prevent backflow of fluids or the like from entering into the air line 228 from the funnel 202a. The air may be used for drying of the body that is exposed within the funnel 202a.

In use, each of the human excrement (e.g., urine), clean water supplied from the water source 218, and air supplied from the air source 226 may be pulled through the hose 206 and directed toward the reclamation system 210. At the fluid separation junction 208, the gaseous and liquid phases of the fluid passing through the hose 206 may be separated, and subsequently treated within the reclamation system 210. In some configurations, the airflow through the hose 206 may also cause liquids and/or gases from the waste container 204 to be extracted therefrom and directed into the reclamation system 210 for processing and/or treatment.

The capture funnel 202 may also include a cover 230 that is arranged to cover and seal the inlet end 212 of the funnel 202a. During use by a human, the cover 230 is removed and the inlet end 212 is open/exposed. After use, the user may place the cover 230 over the inlet end 212 of the funnel 202a. The cover 230 may engage in a liquid-impermeable manner such that any liquid still within the funnel 212 may not exit the capture funnel 200 by way of the inlet end 212. This, in combination with backflow prevention elements on the clean water line 220 and the air line 228, causes any liquids to be directed into and through the hose 206. With the cover 230 in a closed state, the user may perform one or both of the rinsing operation and the drying operation. The cover 230 may also include additional features to be used for cleaning of the funnel 202a. For example, the cover 230 may be equipped with UV light sources or the like to be used before or after the water and/or air cleaning steps, to sanitize surfaces of the funnel 202a.

These operations may serve to clean the funnel 202a. In some embodiments, the water used for the rinsing operation may be pure water or deionized water that is used to ensure a cleaning and/or sanitizing functionality. In other embodiments, the water may be treated in some fashion (e.g., with chemicals or the like) to ensure cleaning, sanitizing, and/or disinfecting of human skin and/or surfaces of the funnel 202a and/or surfaces of the hose 206.

Although described as two separate functions, in some embodiments, a continuous airflow may be driven through the waste management system 200. For example, using a pump or fan, associated with the waste container 204 and/or the hose 206 (e.g., fluid driver 108 shown in FIG. 1), a continuous air suction may be provided to aid in driving both airflow and liquid flow from the funnel 202a, through the hose 206, and into the waste container 204 and/or the reclamation system 210. In some embodiments, the airflow may be continuously run, and the air-function trigger 216 may be used to increase an airflow during the drying operation(s). The air provided during operation of the air-function trigger 216 may be an increase in airflow rate, an increase in air volume, an introduction of treated air (e.g., heated, drying, etc.), or the like.

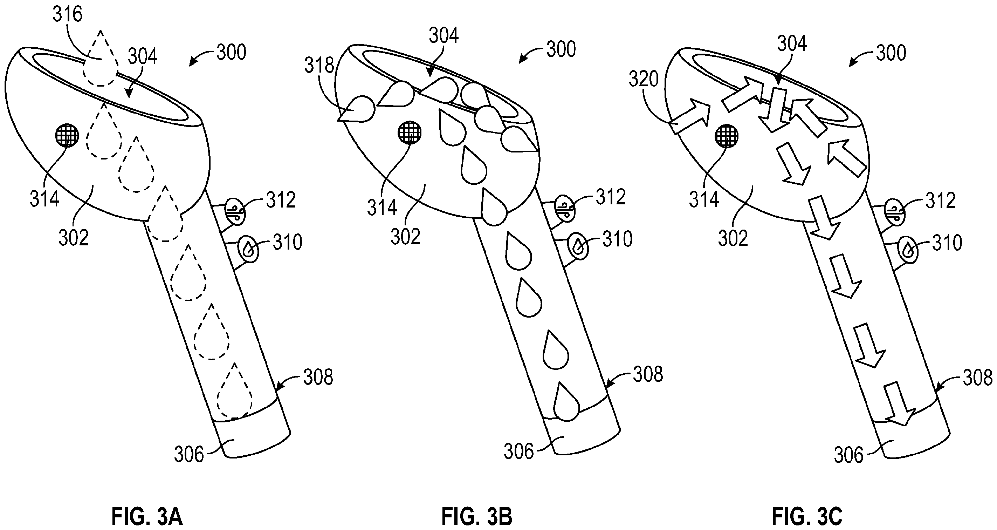

Referring now to FIGS. 3A-3C, schematic illustrations of states of use of a capture funnel 300 of a waste management system in accordance with an embodiment of the present disclosure are shown. The capture funnel 300 may be configured similar to the capture funnel 202 of FIG. 2 and may be used with waste management systems as shown and described herein (e.g., waste management system 100, 200 of FIGS. 1-2).

In FIG. 3A, it is assumed that the capture funnel 300 is being used by a user and is applied and held in place for use by the user. That is, a funnel 302 of the capture funnel 300 is open such that an inlet end 304 is open. When applied and held in place for use, the inlet end 304 may provide a sealing engage with a user's skin or body. The funnel 302 connects to a hose 306 at an outlet end 308 of the funnel 302. The funnel 302 defines an open path or fluid path through the capture funnel 300 from the inlet end 304 to the outlet end 308. The capture funnel 300 includes a water-function trigger 310 and an air-function trigger 312, similar to that shown and described above. The water-function trigger 310 is operably configured to cause clean water to be injected into the funnel 302 from a clean water source (e.g., as shown in FIG. 2). Similarly, the air-function trigger 312 is operably configured to cause air to be injected into the funnel 302 from an air source (e.g., as shown in FIG. 2). Each of the water and air sourced from the respective clean water source and air source may be passed through a tube, hose, pipe, line, or the like that is fluidly coupled to the funnel 302 (e.g., as shown in FIG. 2). Further, each of the lines arranged to supply the respective water or air may include a valve or mechanism to prevent backflow of fluids (e.g., liquid) into the lines.

As shown in FIG. 3A, with the funnel 302 held in place by a user, the user may urinate into the funnel 302, with the waste being directly through the funnel 302 and into the hose 306 and directed downstream to a waste container and/or reclamation system. A continuous pull or directional airflow may pass through the funnel 302 and the hose 306. In some embodiments, an optional airflow aperture 314 may be provided on the funnel 302 to allow for continuous airflow, even when the inlet end 304 of the funnel 302 is held in substantial sealing contact with the body/skin of a user. The airflow aperture 314 may include a filter, membrane, or other feature that is selected to allow for gas to pass through the airflow aperture 314 but prevent liquid from entering or exiting therethrough. As such, only air or a gas may pass through the airflow aperture 314 while liquid may be prevented from entering (or exiting) the funnel 302 through the airflow aperture 314. During use, human waste 316 (e.g., urine) will be directed into the funnel 302 by the user and the force from urinating in combination with a directional flow of air will cause the human waste 316 to be drawn into and through the hose 306.

After urinating into the capture funnel 302 (FIG. 3A), and while the capture funnel 302 is still held in place and in contact with the user, the user may then perform a rinse operation by actuating the water-function trigger 310 (FIG. 3B). By actuating the water-function trigger 310, clean water 318 may be dispensed into the funnel 302, as shown in FIG. 3B. The dispensing of the clean water 318 may be through one or more jets, orifices, apertures, or the like that are fluidly connected to a clean water source. The clean water 318 will impinge on the user's skin and body and aid in removal of any splashed liquid from urinating into the funnel 302. Due to a motive force in the hose 306 (e.g., continuous airflow), and because the funnel 302 is sealed against a user, the clean water 318, after use, will flow into and through the hose 306. Because the clean water 318 was used to clean a user, the water (after use) will be collected in a reclamation system (or waste container), as described above. As noted above, the water rinse cycle may be based on continuous actuation of the water-function trigger 310 (dispenses only when trigger is actuated) or may be set to dispense the clean water 318 for a predetermined a time or other duration (e.g., volume, force, etc.). Further, as noted, even during this step of cleaning, the clean water 318 is prevented from exiting from locations other than the outlet end 308 due to the airflow aperture 314 having a liquid impermeable filter or membrane or the like.

After performing the water cleaning operation (FIG. 3B), and while the capture funnel 302 is still held in place and in contact with the user, the user may then perform a drying operation (FIG. 3C) by actuating the air-function trigger 312. By actuating the air-function trigger 312, air 320 may be dispensed into the funnel 302, as shown in FIG. 3C. The dispensing of the air 320 may be through one or more jets, orifices, apertures, or the like that are fluidly connected to an air source. In some embodiments, the entry locations of the water (FIG. 3B) and the air (FIG. 3C) may be the same, such that a single orifice, jet, aperture (or set thereof) with a connection to each respective line (water line, air line) at the location of the injection. In other embodiments, each of the water and air may be directed into the funnel 302 from separate or dedicated orifices, jets, apertures, or the like. Further, as noted above, the air may be treated before injection or supply into the funnel 302. For example, the air may be heated, dried, or otherwise treated by a heat exchanger, dehumidifier, heater, or the like. The air 320 will impinge on the user's skin and body and aid in removal of any remaining liquid and dry the skin of the user. Due to a motive force in the hose 306 (e.g., continuous airflow), and because the funnel 302 is sealed against a user, the air 320, after use, will flow into and through the hose 306. As noted above, the air drying cycle may be based on continuous actuation of the air-function trigger 312 (dispenses only when trigger is actuated) or may be set to dispense the air 320 for a predetermined a time or other duration (e.g., volume, force, etc.).

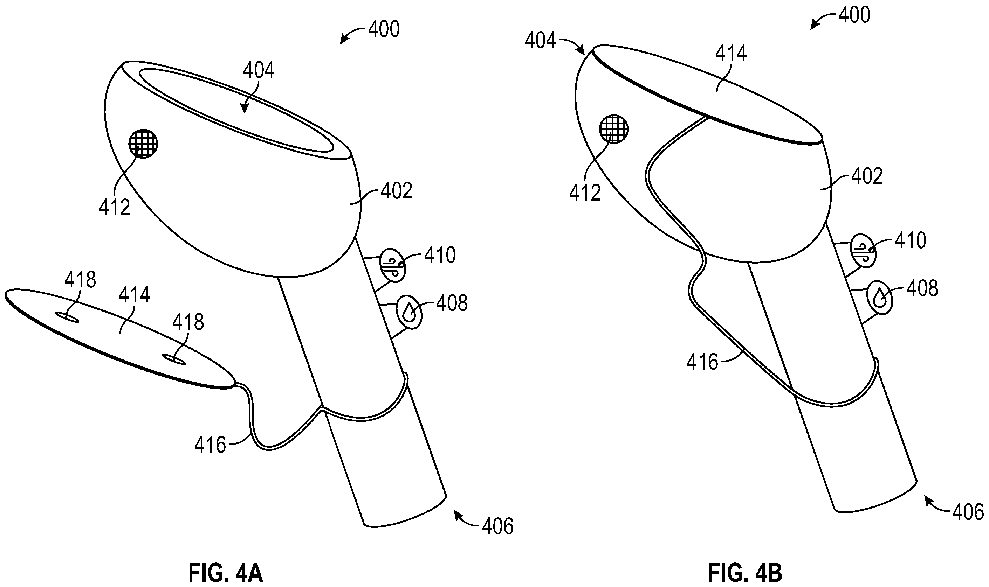

Referring now to FIGS. 4A-4B, schematic illustrations of a capture funnel 400 in accordance with an embodiment of the present disclosure are shown. The capture funnel 400 may be similar to the capture funnels described above. The capture funnel 400 includes a funnel 402 having an inlet end 404 and an outlet end 406. The inlet end 404 defines an opening for use by a user and the outlet end 406 is configured to connect to a hose or the like. The capture funnel 400 is configured to be operated using a water-function trigger 408 and an air-function trigger 410, similar to that described above, and thus the functionality thereof will not be described again. Similar to the embodiments of FIGS. 3A-3C, the funnel 402 includes an optional airflow aperture 412 to allow for airflow into and through the funnel 402 even when the funnel 402 is secured against a user or otherwise sealed against liquids. The airflow aperture 412 may include a filter or membrane or the like that is gas permeable but liquid impermeable.

In this configuration, the capture funnel 400 includes a cover 414 that is arranged to cover and seal the inlet end 404 of the funnel 402. During use by a user, the cover 414 is removed and the inlet end 404 is open/exposed (FIG. 4A). After use, the user may place the cover 414 over the inlet end 404 of the funnel 402. The cover 414 may engage in a liquid-impermeable manner such that any liquid still within the funnel 402 may not exit the capture funnel 400 by way of the inlet end 414. With the cover 414 in a closed state (FIG. 4B), the user may perform one or both of the rinsing operation and the drying operation. In accordance with some embodiments of the present disclosure, the rinse function can include application of a cleaning solution other than water, independently from water use or in combination with a water-based rinsing process. The cover 414 optionally may be connected to the funnel 402 by a tether 416. The tether 416 may be cable, cord, or the like and arranged to ensure that the cover 414 is kept in proximity to the funnel 402. In some embodiments, the tether 416 may be configured to supply electrical power to the cover 414. In other configurations, rather than employing the tether 416, the cover 414 may be connected to the funnel 402 by a hinge or the like. The cover 414 may be configured to sealingly engage with a top surface of the funnel 402 (e.g., at the inlet end). The engagement may be provided by clips, clasps, fasteners, tongue-and-groove, a sliding engagement, or the like. The cover 414 and/or the opening of the funnel 402 at the inlet end 404 may include a gasket or other sealing element to ensure a liquid seal is achieved.

The cover 414 may also include additional features to be used for cleaning of the funnel 402. For example, the cover 414 may be equipped with a sanitizing module 418 to be used before or after the water and/or air cleaning steps, to sanitize surfaces of the funnel 402. FIG. 4A illustrates an underside of the cover 414 to illustrate an example of the sanitizing module 418. In this illustrative embodiment, the sanitizing module includes two UV light sources. In FIG. 4B, the cover 414 is installed such that the UV light sources of the sanitizing module 418 are directed toward the interior of the funnel 402. The sanitizing module 418 may receive electrical power through the tether 416 and/or may be battery powered. Although described as using UV light, it will be appreciated that other mechanisms for sanitizing may be employed without departing from the scope of the present disclosure. For example, and without limitation, in some embodiments, the sanitizing module 418 of the cover 414 may be provided with a source of sanitizing material (e.g., liquid form) housed in a cavity or reservoir within the cover 414 or fluidly connected thereto. The cover 414 may then dispense the sanitizing material into the funnel 402 during a cleaning operation (e.g., when the cover 414 is installed to the funnel 402). In some configurations, the capture funnel 400 may be configured to automatically perform a cleaning/sanitizing operation (including operation of feature of the cover 414) when the cover 414 is secured in place at the inlet end 404 of the funnel 402. Accordingly, the capture funnel 400 may be maintained in a clean state that permits multiple users to use the same capture funnel 400.

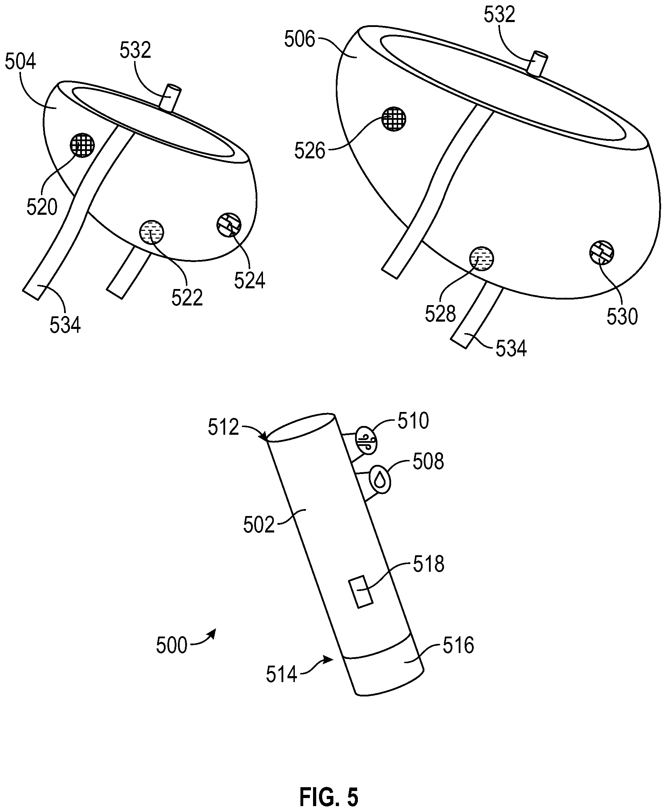

Referring now to FIG. 5, a schematic illustration of a capture funnel 500 in accordance with an embodiment of the present disclosure is shown. In the above illustrated embodiments, the funnel has been generally described and illustrated as a single, unitary structure. However, such configuration is not to be limiting. For example, the capture funnel 500 of FIG. 5 illustrates one configuration in which the capture funnel is separable into distinct parts. For example, the capture funnel 500 includes a stem 502, a first funnel element 504, and a second funnel element 506. The stem 502 includes a water-function trigger 508 and an air-function trigger 510, similar to that shown and described above. The stem 502 has a first end 512 and a second end 514. At the second end 514 of the stem 502 is a hose connector 516 for connection to a hose of a waste management system, as described above. The first end 512 of the stem 502 is configured to receive one or more different funnel elements, such as the first funnel element 504 and the second funnel element 506. The stem 502 may include an optional electrical connector 518 for connection to an electrical power source to supply power to the capture funnel 500 for features thereof (if electrically powered).

Users of the capture funnels described herein may have different anatomies (e.g., male versus female) and/or body types/morphologies, and thus having different size funnel elements may provide for a greater range of use of the capture funnel 500. In this illustration, the capture funnel 500 includes the relatively smaller first funnel element 504 and the relatively larger second funnel element 506. Each funnel element 504, 506 may be selectively connected to the stem 502 to form a capture funnel that is similar to the above described systems. The first funnel element 504 includes an optional airflow aperture 520, a water line connector 522, and an air line connector 524. Similarly, the second funnel element 506 includes an optional airflow aperture 526, a water line connector 528, and an air line connector 530.

The water line connectors 522, 528 are configured to fluidly connect to a water line that, in turn, is connected to a clean water source, as described above. The water line connector 522, 528 may include a valve or the like that keeps the water line connector 522, 528 closed unless a water line is attached thereto. Further, on an interior side (e.g., within the funnel elements 504, 506), the water line connectors 522, 528 may include a nozzle or the like for directing water into the respective funnel elements 504, 506 and at a user (or cover during a cleaning operation). Although shown with a single water line connector 522, 528, it will be appreciated that other water line connectors may be used and/or the single water line connectors 522, 528 may provide a connection point that then distributes the water to one or more nozzles or the like arranged about an interior of the respective funnel elements 504, 506.

The air line connectors 524, 530 are configured to fluidly connect to an air line that, in turn, is connected to an air source, as described above. The air line connector 524, 530 may include a valve or the like that keeps the air line connector 524, 530 closed unless an air line is attached thereto. Further, on an interior side (e.g., within the funnel elements 504, 506), the air line connectors 524, 530 may include a nozzle or the like for directing air into the respective funnel elements 504, 506 and at a user (or cover during a cleaning operation). Although shown with a single air line connector 524, 530, it will be appreciated that other air line connectors may be used and/or the single air line connectors 524, 530 may provide a connection point that then distributes the air to one or more nozzles or the like arranged about an interior of the respective funnel elements 504, 506.

Also shown in FIG. 5, each of the funnel elements 504, 506 include a contact switch 532 on an inlet end surface thereof. The contact switch 532 may be provided to cause opening and use of the various water and air functions only when the contact switch 532 is depressed, such as when the funnel elements 504, 506 are placed in contact with the body of a user or when a cover enclosed the top of the funnel elements 504, 506 and depresses the contact switch 532.

In view of the above, it will be appreciated that various modifications and/or combination may be made to the embodiments disclosed herein. For example, each of the capture funnels 102, 202, 300, 400 may be arranged as shown in FIG. 5, having multiple different funnel elements that are selectively attachable to a stem or the like. Additionally, although shown with the triggers on the stem, it will be appreciated that the trigger(s) may be arranged on the funnel elements, or separate therefrom and connected by a cable or wireless connection. Further, any of the above described embodiments may incorporate the contact switch (e.g., contact switch 532) and/or electrical connector (e.g., electrical connector 518) and/or battery power. In some configurations, the air line and/or continuous air supply into and through the funnel, funnel element, and/or hose, may be automatically started upon picking up of the capture funnel and/or opening of a lid to a waste container (e.g., toilet). Alternatively, a separate operational on/off switch may be provided to ensure continuous airflow operation throughout use of the capture funnels described herein.

An optional harness 534 or the like may be provided with the capture funnel 500, as shown in FIG. 5. As shown, each of the funnel elements 504, 506 includes a respective harness 534. The harness 534 may be a set of adjustable straps, loops, elastic elements, or the like. The harness 534 can be attached at the inlet end of the funnel elements 504, 506 and a user can strap the harness 534 to their body and use the capture funnel 500 with no hands instead of requiring the capture funnel 500 to be held in place by hand. It will be appreciated that the harness 534 may also be used to ensure a complete seal and contact between the funnel elements 504, 506 and the user's body. It will be appreciated that harness 534 shown in FIG. 5 may be implemented in any of the above described embodiments and variations thereon.

Advantageously, embodiments described herein provide for improved waste management systems. In accordance with some embodiments of the present disclosure, waste management systems may be provided with improved capture funnels for use by users to reduce issues associated with waste capture. Embodiments of the present disclosure may provide for improved product and use life of hoses and other components associated with waste management systems. One such feature to improve product life is a built-in cleaning function provided by a cover and water and air lines and dispensing systems for cleaning a funnel and hose of the waste management system. Further, advantageously, due to such cleaning functionality, it may not be necessary to remove the funnel between urination and cleaning, thus reducing odor and pathogen transmission and increasing cleanliness and sanitation. Furthermore, due to the built-in cleaning capability to be applied to a user, embodiments of the present disclosure may eliminates consumables for wiping after use, and thus reduce total waste generation.

Further, advantageously, embodiments of the present disclosure provide for a handheld or manually operated funnel bidet that may decrease high consumable usage and decrease messes associated therewith. Further benefits of the disclosed embodiments include that the waste container will be able to be filled with more waste deposits instead of used consumables, which in turn can extend the life of the waste container. Additionally, the funnel bidets disclosed herein can provide less risk of urine contamination to a cabin or other habitable space/area as there is no exposure time between urination and cleaning oneself (or the funnel itself). This can decrease the risk of pathogen transmission. Further, the self-cleaning feature can allow for multiple users to safely use a clean funnel between uses and keep the urine hose clean, extending the life of the system.

The use of the terms "a", "an", "the", and similar references in the context of description (especially in the context of the following claims) are to be construed to cover both the singular and the plural, unless otherwise indicated herein or specifically contradicted by context. The modifier "about" used in connection with a quantity is inclusive of the stated value and has the meaning dictated by the context (e.g., it includes the degree of error associated with measurement of the particular quantity). All ranges disclosed herein are inclusive of the endpoints, and the endpoints are independently combinable with each other.

While the present disclosure has been described in detail in connection with only a limited number of embodiments, it should be readily understood that the present disclosure is not limited to such disclosed embodiments. Rather, the present disclosure can be modified to incorporate variations that fall within the scope of the invention as defined by the claims.

## Claims

1. A capture funnel for use with a waste management system, the capture funnel comprising:
a funnel (202a) having an inlet end (212) and an outlet end (308);
a water-function trigger (214) actuatable to dispense water into the funnel; and
an air-function trigger (216) actuatable to dispense air into the funnel.

2. The capture funnel of claim 1, wherein the funnel comprises a funnel element (504) and a stem (502), wherein the funnel element is configured to selectively attach and detach from the stem.

3. The capture funnel of claim 2, wherein the funnel element is a first funnel element and the capture funnel comprises a second funnel element (506), wherein the second funnel element is different in size from the first funnel element, and wherein each of the first funnel element and the second funnel element are configured to selective attach and detach from the stem.

4. The capture funnel of any preceding claim, further comprising a cover (230) configured to selectively and sealing engage with the inlet end of the funnel, and optionally wherein the cover comprises a sanitizing module (418) configured to sanitize an interior of the funnel when the cover is engaged with the inlet end of the funnel.

5. The capture funnel of any preceding claim, further comprising a hose adapter at the outlet end of the funnel, wherein the hose adapter is configured to sealing connect to a hose of a waste management system.

6. A waste management system comprising:
a waste container (104);
a reclamation system (118);
a capture funnel (102) as claimed in any preceding claim; and
a hose (106) fluidly connecting the capture funnel to each of the waste container and the reclamation system.

7. The waste management system of claim 6, wherein the funnel comprises at least one airflow aperture (314) arranged to permit gases to pass therethrough and prevent liquid from passing through the at least one airflow aperture.

8. The waste management system of claim 6 or 7, further comprising a clean water source fluidly connected to the funnel by a water line, wherein actuation of the water-function trigger causes clean water from the clean water source to be dispensed into the funnel.

9. The waste management system of claim 8, further comprising a water pump (222) arranged along the water line and is selectively operable based on actuation of the water-function trigger.

10. The waste management system of claim 8 or 9, further comprising a valve (224) arranged between an end of the water line and the funnel, wherein the valve is configured to prevent backflow of liquid into the water line from the funnel.

11. The waste management system of any of claims 6 to 10, further comprising an air source (226) fluidly connected to the funnel by an air line (228), wherein actuation of the air-function trigger causes air from the air source to be dispensed into the funnel, and optionally further comprising a valve arranged between an end of the air line and the funnel, wherein the valve is configured to prevent backflow of liquid into the air line from the funnel

12. The waste management system of claim 11, further comprising an air treatment module (227) arranged along the air line and configured to treat the air prior to being dispensed into the funnel, and optionally wherein the air treatment module comprises at least one of a heater and a dehumidifier.

13. The waste management system of any of claims 6 to 12, further comprising a fluid separation junction (208) arranged along the hose and configured to separate liquid and gas fluids passing through the hose.

14. The waste management system of any of claims 6 to 13, wherein the waste container comprises a removable container insert (112).

15. The waste management system of any of claims 6 to 14, wherein the waste management system is configured for use in a low gravity environment.
